# EUROPEAN PATENT APPLICATION

(11) **EP 3 406 281 A1**
(43) Date of publication of application: **28.11.2018**
(21) Application number: 17741427.3
(22) Date of filing: 18.01.2017
(51) Int. Cl.: A61M 5/31, A61J 1/05, A61K 9/08, A61K 38/00, A61M 5/28

(54) **SYNTHETIC-RESIN OUTER CYLINDER FOR SYRINGE, SYRINGE, PREFILLED SYRINGE, AND LIQUID-FILLED STERILIZED SYNTHETIC-RESIN CONTAINER**

(30) Priority: 20.01.2016 JP 2016008960
(71) Applicant: Terumo Kabushiki Kaisha, Tokyo 151-0072 (JP)
(72) Inventor: MARUYAMA, Sayaka, Ashigarakami-gun Kanagawa 259-0151 (JP); ABE, Yoshihiko, Ashigarakami-gun Kanagawa 259-0151 (JP)
(74) Representative: Casalonga
(86) International application number: PCT/JP2017/001542
(87) International publication number: WO 2017/126550

(57) **Abstract**

A synthetic-resin outer cylinder (2) for a syringe has an open distal-end portion or an injection needle fixed to a distal-end portion thereof and is sterilized by radioactive rays or electron beams. The synthetic resin forming the outer cylinder contains a phenol-based antioxidant. The amount of phenoxyl radicals of the synthetic resin measured by the electron spin resonance apparatus is 0.1 to 1.0 × 10¹² spins/mg.

## Description

### TECHNICAL FIELD

The present invention relates to a synthetic-resin outer cylinder for a syringe, the syringe, a prefilled syringe, and a liquid-filled sterilized synthetic-resin container.

### BACKGROUND ART

As a container for a liquid and an outer cylinder for a syringe, those made of glass are mostly used. Glass is disadvantageous in that it is heavy and fragile. Plastic containers and plastic syringes have been developed and are in widespread use.

But in medical applications, there have been cases in which effective components of a liquid, for medical use, accommodated inside the plastic container may be oxidatively denatured. As plastic materials to be used to produce containers for medical use, polypropylene, polyethylene, cyclic olefin polymers, polyvinyl chloride, polyester, polyamide, polycarbonate, and polymethacrylate are exemplified.

As a prior art of accommodating a protein solution formulation inside a container made of the cyclic olefin-based polymer, the container made of the cyclic olefin-based polymer disclosed in patent document 1 (Japanese Patent Application Laid-Open Publication No. 2014-51502 (USP 7253142, EP Application Publication No. 1232753) accommodates genetic recombination protein containing sugar chains. As another prior art, the container made of the cyclic olefin-based polymer disclosed in patent document 2 (Japanese translation of PCT International Application Publication No. 2001-506887, WO 98-27925) accommodates active medical agents such as insulin, human growth hormone, and the like. As still another prior art, the container made of the cyclic olefin-based polymer disclosed in patent document 3 (Japanese Patent Application Laid-Open Publication No. 2003-113112) accommodates sterile calcitonins.

In the protein solution formulation in container, it is necessary to sterilize the container accommodating the protein solution formulation. Because protein is coagulated or denatured when it is heated, it is impossible to sterilize the protein solution formulation by heating the container, for example by autoclaving the container after the protein solution formulation is accommodated inside the container. Thus, in the case of the protein solution formulation contained in the container, after the container is sterilized, the protein solution formulation prepared aseptically is filled in the container. As a method of sterilizing the container before the protein solution formulation is filled therein, it is normal to adopt a method of irradiating the container by radioactive rays (γ) or electron beams. Recently, sterilization of the container by radioactive rays (γ) or electron beams with a medical agent being filled therein is in consideration.

### PRIOR ART DOCUMENTS

### PATENT DOCUMENTS

Patent document 1: Japanese Patent Application Laid-Open Publication No. 2014-51502 (USP 7253142, EP Application Publication No. 1232753)
Patent document 2: Japanese translation of PCT International Application Publication No. 2001-506887 (WO 98-27925)
Patent document 3: Japanese Patent Application Laid-Open Publication No. 2003-113112

### SUMMARY OF THE INVENTION

### PROBLEMS TO BE SOLVED BY THE INVENTION

In a case where the container is sterilized by radioactive rays (γ) or electron beams as described above, there is a case in which effective components of a liquid, for medical use, accommodated inside the plastic container are oxidatively denatured.

As a result of present inventors' extensive examinations, they have obtained the following knowledge: The above-described resin and the additive such as an antioxidant to be added to the resin do not cause the effective components of the liquid to be oxidatively denatured before the container is sterilized. But when containers and outer cylinders formed of the above-described resin are sterilized by radioactive rays or electron beams, the effective components contained in the liquid for medical use are oxidatively denatured.

As a result of the present inventors' detailed investigations, they have checked and confirmed that by sterilizing the containers and the outer cylinders by radioactive rays or electron beams, phenoxyl radicals are generated from a phenol-based antioxidant added to synthetic resin. They have found that the phenoxyl radicals are the substance which causes the effective components of the liquid to be oxidatively denatured. But the phenol-based antioxidant is generally used as an antioxidant for synthetic resin and has a good antioxidant effect. Thus, it is difficult that the synthetic resin does not contain the phenol-based antioxidant from the standpoint of stability of the container made of the synthetic resin.

It is an object of the present invention to provide a synthetic-resin outer cylinder for a syringe, which contains a phenol-based antioxidant and causes a medical agent filled inside the outer cylinder to be oxidatively denatured to an extremely low extent, although the outer cylinder is sterilized by radioactive rays or electron beams, the syringe, a prefilled syringe, and a liquid-filled sterilized synthetic-resin container.

### MEANS FOR SOLVING THE PROBLEMS

The synthetic-resin outer cylinder for the syringe, which achieves the above-described object of the present invention has the following form:
The synthetic-resin outer cylinder for a syringe is sterilized by radioactive rays or electron beams. The synthetic-resin outer cylinder has an open distal-end portion or an injection needle fixed to a distal-end portion thereof. The synthetic resin contains a phenol-based antioxidant. The amount of phenoxyl radicals of the synthetic resin measured by an electron spin resonance apparatus is 0.1 to 1.0 × 10¹² spins/mg.

The syringe which achieves the above-described object of the present invention has the following form:
The syringe has the above-described synthetic-resin outer cylinder, a gasket slidable inside the outer cylinder, and a plunger mounted on the gasket. The syringe is sterilized by radioactive rays or electron beams together with the outer cylinder.

The prefilled syringe which achieves the above-described object of the present invention has the following form:
The prefilled syringe has a synthetic-resin outer cylinder which has an open distal-end portion or an injection needle fixed to a distal-end portion thereof, a gasket slidable inside the outer cylinder, a plunger mounting on the gasket, a sealing member sealing the open distal-end portion of the synthetic-resin outer cylinder or the injection needle, and a liquid for medical use filled inside the outer cylinder. The prefilled syringe is sterilized by radial rays or electron beams. The synthetic-resin outer cylinder contains a phenol-based antioxidant. The amount of phenoxyl radicals measured of the synthetic-resin outer cylinder by an electron spin resonance apparatus is 0.1 to 1.0 × 10¹² spins/mg. The liquid for medical use contains substances, for medical use, which undergo oxidative denaturation.

The synthetic-resin container which achieves the above-described object of the present invention has the following form:
The liquid-filled sterilized synthetic-resin container has a synthetic-resin container body, a sealing member sealing an open portion of the container body, and a liquid for medical use accommodated inside the container body. The synthetic-resin container is sterilized by radioactive rays or electron beams. The synthetic-resin container body contains a phenol-based antioxidant. The amount of phenoxyl radicals of the synthetic-resin container body measured by an electron spin resonance apparatus is 0.1 to 1.0 × 10¹² spins/mg. The liquid for medical use contains substances, for medical use, which undergo oxidative denaturation.

### BRIEF DESCRIPTION OF THE DRAWINGS

Fig. 1 is a front view of a synthetic-resin outer cylinder of the present invention for a syringe.
Fig. 2 is a vertical sectional view of the synthetic-resin outer cylinder for the syringe shown in Fig. 1.
Fig. 3 is a front view of a prefilled syringe of one embodiment of the present invention.
Fig. 4 is a sectional view taken along a line A-A of Fig. 3.
Fig. 5 is a front view of a liquid-filled sterilized synthetic-resin container of the present invention.
Fig. 6 is a sectional view taken along a line B-B of Fig. 5.
Fig. 7 is a front view of a prefilled syringe of another embodiment of the present invention.
Fig. 8 is a sectional view taken along a line C-C of Fig. 7.

### MODE FOR CARRYING OUT THE INVENTION

A synthetic-resin outer cylinder of the present invention for a syringe and a prefilled syringe of the present invention are described below with reference to embodiments shown in the drawings.

A synthetic-resin outer cylinder 2 of the present invention for the syringe has an open distal-end portion or an injection needle fixed to a distal-end portion thereof and is sterilized by radioactive rays or electron beams. The synthetic resin forming the outer cylinder contains a phenol-based antioxidant. The amount of phenoxyl radicals of the synthetic resin measured by an electron spin resonance apparatus is 0.1 to 1.0 × 10¹² spins/mg.

The syringe of the present invention has the outer cylinder 2 made of synthetic resin, a gasket 4 slidable inside the outer cylinder 2, and a plunger 5 mounted on the gasket 4. The syringe is entirely sterilized by radioactive rays or electron beams together with the outer cylinder 2.

The outer cylinder 2 of this embodiment for the syringe has an outer cylinder body part 21, a nozzle part 22 provided at a distal end side of the outer cylinder body part 21, and a flange 23, provided at a proximal end of the outer cylinder body part 21, which projects outward.

The synthetic-resin outer cylinder 2 of the present invention for the syringe contains the phenol-based antioxidant. The amount of the phenoxyl radicals of the synthetic-resin outer cylinder measured by the electron spin resonance apparatus is 0.1 to 1.0 × 10¹² spins/mg. The outer cylinder 2 for the syringe is a tubular body formed of a transparent material or a semitransparent material which has preferably a low oxygen permeability and a low water vapor permeability.

As materials for forming the outer cylinder 2, it is possible to use olefin-based resin including polyolefin such as low-density polyethylene, medium density polyethylene, high-density polyethylene, linear low-density polyethylene, linear ultra-low-density polyethylene, polypropylene, poly-1-butene, poly-4-methyl-1-pentene or random or block copolymers of α-olefins such as ethylene, propylene, 1-butene, 4-methyl-1-pentene and the like, and acid-modified polyolefin such as maleic anhydride grafted polyethylene, maleic anhydride grafted polypropylene, and the like; ethylene-vinyl compound copolymers such as ethylene-vinyl acetate copolymers, ethylene-vinyl alcohol copolymers, ethylene-vinyl chloride copolymers, ethylene-(meta) acrylic acid copolymers, and ion-crosslinked products thereof (ionomers), ethylene-methyl methacrylate copolymers, and the like; styrene-based resin such as polystyrene, acrylonitrile-styrene copolymers, α-methyl styrene-styrene copolymers, and the like; polyvinyl compounds such as polymethyl acrylate, polymethyl methacrylate, and the like; polyamide such as nylon 6, nylon 66, nylon 610, nylon 12, nylon 6IT, and poly(meta-xylylene adipamide (MXD6); polyester such as polyethylene terephthalate (PET), polybutylene terephthalate (PBT), polytrimethylene terephthalate (PTT), polyethylene naphthalate (PEN), glycol modified polyethylene terephthalate (PETG), polyethylene succinate (PES), polybutylene succinate (PBS), polylactic acid, polyglycolic acid, polycaprolactone, and polyhydroxyalkanoate; copolymers, resulting from norbornene and olefin such as ethylene, which include polyether such as polycarbonate, polyethylene oxide, and the like; cycloolefin copolymers (COC) resulting from tetracyclo-dodecene and olefin such as ethylene, and cycloolefin polymers (COP) which is a polymer produced by performing ring-opening polymerization of norbornene and by performing hydrogenation and the like or mixtures of these polymers.

As the phenol-based antioxidant, hindered phenol-based antioxidants are suitable. As the phenol-based antioxidant, it is possible to preferably use the hindered phenol-based antioxidants such as BHT, 2,2'-methylenebis (4-methyl-6-tert-butylphenol), pentaerythritol tetrakis [3-(3,5-di-tert-butyl-4-hydroxyphenyl) propionate], 3,3',3",5,5',5"-hexa-tert-butyl-α,α',α"-(mesitylene-2,4,6-triyl) tri-p-cresol, octadecyl-3-(3,5-di-tert-butyl-4-hydroxyphenyl)propionate, 1,3,5-tris[(4-tert-butyl-3-hydroxy-2,6-xylyl)methyl]-1,3,5-triazine-2,4,6(1H,3H,5H)-trione, 1,3,5-tris[(3,5-di-tert-butyl-4-hydroxybenzyl-1,3,5 triazine-2,4,6(1H,3H,5H)-trione, calcium diethyl bis[{3,5-bis(1,1-dimethylethyl)-4-hydroxyphenyl}methyl]phosphonate, bis(2,2'-dihydroxy-3,3'-di-tert-butyl-5,5'-dimethylphenyl)ethane, and NN'-hexane-1,6-diylbis[3-(3,5-di-tert-butyl)-4-hydroxyphenyl]propionamide, and the like. As examples of hindered phenol compounds commercially available, Irganox 1010 (trade name) and Irganox 1098 (trade name) produced by BASF Corporation are exemplified.

The synthetic resin may contain the phenol-based antioxidant and an antioxidant not phenol-based. As the antioxidant not phenol-based, it is possible to use a phosphorous-based antioxidant, an aromatic amine-based antioxidant, a hindered amine-based antioxidant, and the like.

Examples of the phosphorous-based antioxidant include organic phosphorous compounds such as triphenyl phosphite, trioctadecyl phosphite, tridecyl phosphite, tri nonylphenyl phosphite, diphenyl isodecyl phosphite, bis(2,6-di-tert-butyl-4-methylphenyl)pentaerythritol diphosphite, bis(2,4-di-tert-butylphenyl) pentaerythritol diphosphite, tris(2,4-di-tert-butylphenyl) phosphite, distearyl pentaerythritol diphosphite, tetra(tridecyl-4,4'-isopropylidene diphenyl diphosphite, and 2,2-methylene-bis(4,6-di-tert-butylphenyl)octyl phosphite. These organic phosphorous compounds can be used singly or as mixtures thereof.

As the aromatic amine-based antioxidant, phenyl naphthylamine, 4,4'-dimethoxydiphenylamine, 4,4'-bis(α, α-dimethylbenzyl)diphenylamine, and 4-isopropoxydiphenylamine are exemplified. As the hindered amine-based antioxidant, 2-(3,5-di-t-butyl-4-hydroxybenzyl)-2-n-butylmalonic acid bis(1,2,2,6,6-pentamethyl-4-piperidyl) (TINUVIN (registered trademark) 144 produced by BASF Corporation) is exemplified.

The total content of the antioxidant contained in the material for forming the outer cylinder 2 is favorably 0.01 to 2 wt% and especially favorably 0.05 to 1 wt%. The content of the phenol-based antioxidant contained in the material for forming the outer cylinder 2 is favorably 0.0001 to 0.002 wt% and especially favorably 0.0005 to 0.001 wt%.

The material for forming the outer cylinder 2 may contain additives such as a lubricant, a delustering agent, a heat stabilizer, a weathering stabilizer, an ultraviolet absorber, a plasticizer, a flame retardant, an antistatic agent, an anti-coloring agent, and a crystallization nucleating agent.

The synthetic-resin outer cylinder of the present invention is sterilized by radioactive rays or electron beams. It is favorable to sterilize the outer cylinder by electron beams. The amount of the phenoxyl radicals contained in the synthetic-resin outer cylinder 2 sterilized by radioactive rays or electron beams is 0.1 to 1.0 × 10¹² spins/mg when the amount thereof is measured by the electron spin resonance apparatus. Therefore, although the synthetic-resin outer cylinder for the syringe contains the phenol-based antioxidant and is sterilized by radioactive rays or electron beams, the outer cylinder allows a medical agent filled therein to be oxidatively denatured to a very low extent. The phenoxyl radicals are generated when the phenol-based antioxidant is radicalized. The phenoxyl radicals are generated by radicalization of the phenol-based antioxidant when synthetic resin containing the phenol-based antioxidant is irradiated by radioactive rays or electron beams. The amount of the phenoxyl radicals contained in the synthetic-resin outer cylinder 2 is preferably 0.1 to 0.3 × 10¹² spins/mg when the amount thereof is measured by the electron spin resonance apparatus.

The outer cylinder 2 has the outer cylinder body part 21, the nozzle part 22 provided at the distal end side of the outer cylinder body part 21, and the flange 23 provided at the proximal end side of the outer cylinder body part 21.

The outer cylinder body part is a substantially tubular part accommodating the gasket 4 liquid-tightly and slidably. The nozzle part is formed as a tubular part having a smaller diameter than the outer cylinder body part 21. A distal-end portion of the outer cylinder body part is formed as a tapered portion whose diameter decreases toward the nozzle part.

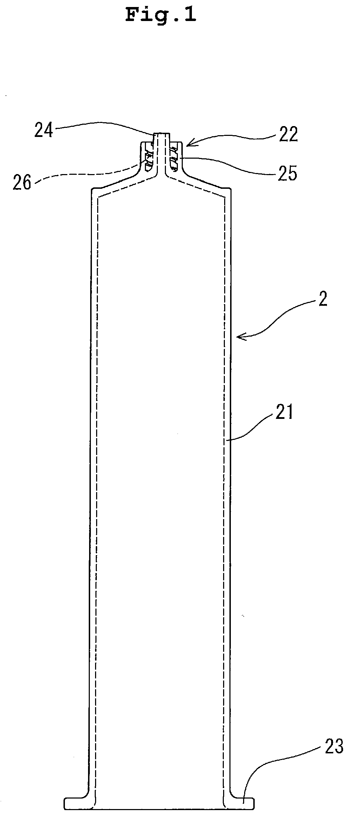

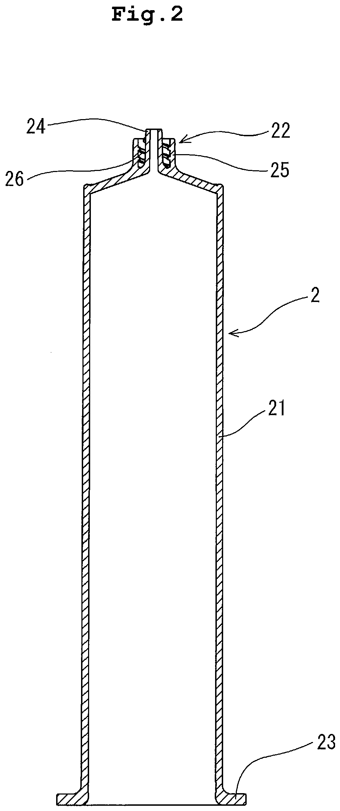

As shown in Figs. 1 and 2, the flange 23 has an arc-shaped outer edge formed by projecting the flange from the entire circumference of the proximal end of the outer cylinder body part 21 at an angle vertical to the outer cylinder body part. In other words, the flange has a shape of a doughnut plate whose inner portion is omitted.

As shown in Figs. 1 and 2, the nozzle part 22 has a nozzle body portion 24 and a collar 25 formed concentrically with the nozzle body portion 24. The nozzle body portion 24 is provided at a distal end of the outer cylinder 2. The nozzle body portion has a distal-end open portion for discharging a liquid medicine or the like contained inside the outer cylinder and is so formed that its diameter decreases toward its distal end in a tapered shape. The collar 25 is formed cylindrically and concentrically with the nozzle part 22 in such a way as to surround the nozzle part 22. The collar is open at its distal end. The inner and outer diameters of the collar 25 are substantially equal to each other from its proximal end to distal end. A distal-end portion of the nozzle body portion 24 is projected beyond a distal-end opening of the collar 25. The distal-end portion of the nozzle body portion 24 and that of the collar 25 are chamfered so that the nozzle body portion 24 and the collar 25 can be easily accommodated inside a sealing member (seal cap) 3.

At the inner circumferential surface of the collar 25, a screw groove (threadedly engaging portion at outer cylinder side) 26 which engages a rib formed at a nozzle accommodation part of a sealing member (seal cap) 3 which is described later and a hub (not shown) of an injection needle to be connected thereto at the time of use. Thereby the outer cylinder 2 and the seal cap 3 engage with each other between the inner circumferential surface of the collar and the outer circumferential surface of the nozzle accommodation part. The injection needle (hub of injection needle) is mounted on the screw groove (threadedly engaging portion at outer cylinder side) 26 after the seal cap 3 is removed from the outer cylinder.

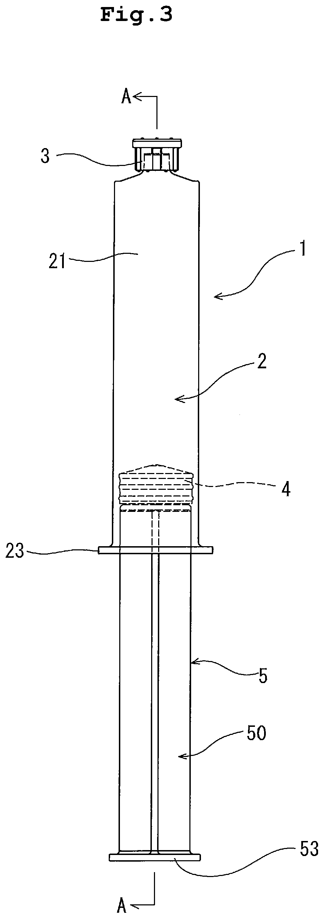

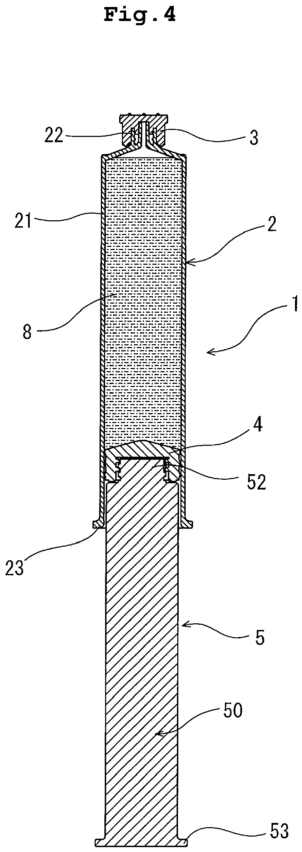

As shown in Figs. 3 and 4, the gasket 4 has a tubular body part extended in a substantially equal outer diameter and a tapered closed part extended from its body part to its distal end. A plurality of annular ribs (in this embodiment, three ribs are formed. When two or more annular ribs are formed, the number thereof may be appropriately selected, provided that liquid tightness and slidability are satisfied)) is formed on an outer surface of the body part. These ribs contact an inner surface of the outer cylinder 2 liquid-tightly. The closed part of the gasket 4 has a configuration corresponding to that of the inner surface of the distal end of the outer cylinder 2 to prevent a gap from being formed as much as possible between the closed part of the gasket and the inner surface of the distal end of the outer cylinder when the closed part of the gasket is brought into contact with the inner surface of the distal end of the outer cylinder 2.

The gasket 4 has a concave portion, disposed inside the tubular body part, which is extended from a proximal-end open portion of the tubular body part to a distal end thereof. The concave portion is capable of accommodating the mounting distal-end portion 52 of the plunger 5 therein. A gasket-side threadedly engaging portion is formed on an inner surface (inner surface of tubular body part) of the concave portion. The gasket-side threadedly engaging portion is capable of threadedly engaging with a plunger-side threadedly engaging portion formed on an outer surface of the mounting distal-end portion 52 formed at a distal-end portion of the plunger 5. The plunger 5 does not separate from the gasket 4 owing to threaded engagement between the gasket-side threadedly engaging portion and the plunger-side threadedly engaging portion. It is possible to remove the plunger 5 from the gasket and mount the former on the latter at the time of use. A plurality of ribs is formed on a lower-end surface of the tubular body part of the gasket 4.

As materials for forming the gasket 4, it is preferable to use elastic rubber (for example, butyl rubber, Latex rubber, silicone rubber) and synthetic resin (for example, a styrene-based elastomer such as an SBS elastomer, an SEBS elastomer; and an olefin-based elastomer such as an ethylene-a olefin copolymer elastomer).

The plunger 5 has a plunger body part 50 and the mounting distal-end portion 52, to be mounted on the gasket 4, which is projected from the plunger body part 50 to the distal end thereof. The plunger body part 50 has a shaft portion formed in a cross shape in its cross section and a pressing portion 53 provided at a proximal end of the shaft portion.

The shaft portion of the plunger body part 50 is formed of four flat plate portions. A distal end of the plunger body part 50 (shaft portion) is provided with a flange. A proximal-end portion of the plunger body part 50 is provided with the disk-shaped pressing portion 53.

The mounting distal-end portion 52 is a projected part provided at a distal-end portion of the plunger 5. The mounting distal-end portion 52 is projected forward (toward distal end) from a vicinity of the center of the flange. It is desirable that the mounting distal-end portion 52 is columnar or tubular.

The plunger-side threadedly engaging portion which threadedly engages the gasket-side threadedly engaging portion of the gasket 4 is provided on the outer surface of the mounting distal-end portion 52. The plunger-side threadedly engaging portion is formed of spiral ribs. The plunger-side threadedly engaging portion has two spiral ribs in correspondence with the spiral threadedly engaging portion of the gasket 4. The number of the spiral ribs may be one. In the syringe of this embodiment, by rotating the plunger 5, the plunger 5 is mounted on the gasket 4, as will be described later.

As materials for composing the plunger 5, it is preferable to use hard or semi-hard resin such as high-density polyethylene, polypropylene, polystyrene, polyethylene terephthalate, and the like.

The syringe is sterilized by radioactive rays or electron beams with the outer cylinder 2, the gasket 4, and the plunger 5, all of which are component parts of the syringe being accommodated inside one package. In a case where the outer cylinder 2 made of synthetic resin is sold alone, the outer cylinder is sterilized by radioactive rays or electron beams with one or more of the outer cylinders being accommodated inside the package.

A prefilled syringe 1 of the present invention is composed of the synthetic-resin outer cylinder 2 which has the open distal-end portion of the outer cylinder or an injection needle forming a distal-end portion of the outer cylinder, the gasket 4 slidable inside the outer cylinder 2, the plunger 5 mounted on the gasket 4, the sealing member 3 sealing the open distal-end portion of the outer cylinder 2 or the injection needle, and the liquid for medical use filled inside the outer cylinder. The prefilled syringe is sterilized by radial rays or electron beams. The synthetic-resin outer cylinder 2 contains the phenol-based antioxidant. The amount of the phenoxyl radicals of the synthetic-resin outer cylinder 2 measured by the electron spin resonance apparatus is 0.1 to 1.0 × 10¹² spins/mg. The liquid 8 for medical use contains substances, for medical use, which undergo oxidative denaturation.

As the outer cylinder 2, the gasket 4, and the plunger 5, those described above are used.

In the prefilled syringe 1 of the present invention, the liquid 8 for medical use filled inside the outer cylinder contains substances, for medical use, which undergoes oxidative denaturation. A protein solution formulation is a typical example of the liquid for medical use containing the substances, for medical use, which undergo oxidative denaturation.

The protein solution formulation which contains protein having a methionine residue and is biologically active and which is preferably used in a medical field is preferably used. As examples of the protein solution formulation, it is possible to list solution formulations containing hematopoietic factors such as erythropoietin, granulocyte colony stimulating factor, granulocyte macrophage colony stimulating factor, and thrombopoietin; molecular targeted drugs such as cytokines, monoclonal antibody, and the like; and proteins such as serum albumin, tissue plasminogen activator, stem cell growth factor, interferon, and interleukin.

The methionine residue is especially oxidizable in proteinogenic amino acid residues. A cysteine residue forms a disulfide crosslinking intramolecularly or intermolecularly when it is oxidized, thus influencing a high-order structure of protein. Therefore, of the proteins exemplified above, the container for medical use of the present invention is suitable for accommodating solution formulations containing proteins having the methionine residue or the cysteine residue in the sequence of amino acids such as erythropoietin, abatacept, etanercept, adalimumab, rituximab, trastuzumab, and palivizumab.

In the molecularly targeted drug consisting of the protein such as the monoclonal antibody, the high-order structure of the protein is especially important to allow it to be effective as a medical agent. Thus, the container of the present invention for medical use is especially suitable for accommodating the solution formulation containing the molecularly targeted drug consisting of the proteins having the methionine residue or the cysteine residue in the sequence of the amino acids such as abatacept, etanercept, adalimumab, rituximab, trastuzumab, and palivizumab.

The mixing content, pH, and other properties of the protein solution formulation to be accommodated inside the container of the present invention for medical use are not specifically limited, but in dependence on the kind and the like of the protein solution formulation, it is possible to set the mixing content and properties hitherto adopted in each protein solution formulation.

The protein solution formulation to be accommodated inside the container of the present invention for medical use may contain one kind or two or more kinds of a stabilizer, a buffer, a solubilizing agent, a tonicity agent, a pH adjustor, a soothing agent, a reducing agent, an antioxidant, and other components.

As stabilizers the protein solution formulation is capable of containing, it is possible to exemplify surface active agents including nonionic surface active agents (sorbitan fatty acid ester, glycerin fatty acid ester, polyglycerin fatty acid ester, polyoxyethylene sorbitan fatty acid ester, polyoxyethylene sorbit fatty acid ester, polyoxyethylene glycerin fatty acid ester, polyethylene glycol fatty acid ester, polyoxyethylene alkyl ether, polyoxyethylene polyoxypropylene alkyl ether, polyoxyethylene alkyl phenyl ether, polyoxyethylene hardened castor oil, polyoxyethylene beeswax derivatives, polyoxyethylene lanolin derivatives, polyoxyethylene fatty acid amide, lecithin, glycerophospholipid, sphingophospholipid, and sucrose fatty acid ester and the like) and anionic surface active agents (alkyl sulfates, polyoxyethylene alkyl ether sulfates, and alkyl sulfosuccinates and the like); and amino acids and the like.

Of these stabilizers, the polyoxyethylene sorbitan fatty acid ester is favorable. Polyoxyethylene sorbitan monooleate (polysorbate 80) and/or polyoxyethylene sorbitan monolaurate (polysorbate 20) are more favorable.

As examples of the amino acids to be used as the stabilizer, it is possible to exemplify leucine, tryptophan, serine, glutamic acid, arginine, histidine, lysine, methionine, phenylalanine, acetyl tryptophan, and salts of these amino acids. The amino acid may consist of any of an L-body, a D-body, and a DL-body.

Of these amino acids, L-leucine, L-tryptophan, L-glutamic acid, L-arginine, L-histidine, L-lysine, and salts of these amino acids are preferably used.

As the buffer, it is possible to exemplify phosphates such as dibasic sodium phosphate, sodium dihydrogen phosphate, and citrates such as sodium citrate.

As the solubilizing agent, it is possible to exemplify the polyoxyethylene sorbitan monooleate (polysorbate 80) and/or polyoxyethylene sorbitan monolaurate (polysorbate 20), cremophor, ethanol, and sodium dodecylbenzene sulfonate.

As the tonicity agent, it is possible to exemplify sugars such as polyethylene glycol, dextran, mannitol, sorbitol, inositol, glucose, fructose, lactose, xylose, mannose, maltose, sucrose, and raffinose.

The content of the protein contained in the protein solution formulation to be accommodated inside the container of the present invention for medical use is not specifically limited, but it is possible to adjust the content thereof in dependence on the kind of the protein and the application and use form of the protein solution formulation.

The liquid for medical use containing the substances, for medical use, which undergo oxidative denaturation is not limited to the protein solution formulation, but biopharmaceuticals and the like are exemplified. For example, the biopharmaceutical means a medical agent produced by using a biotechnology such as a cell culture technique or a gene recombination technique. Protein biopharmaceuticals, nucleic acid drugs, and peptide drugs, and the like are exemplified. More specifically, examples of the biopharmaceutical include various monoclonal antibodies, various vaccines, interferons, insulin, growth hormone, erythropoietin, colony stimulating factors, TPA, interleukin, a coagulation VIII factor, a coagulation IX factor, natriuretic hormone, somatomedin, glucagon, serum albumin, calcitonin, a growth hormone releasing factor, a digestive enzyme agent, an inflammatory enzyme agent, antibiotics, antisense nucleic acid, antigen nucleic acid, decoy nucleic acid, aptamer, siRNA, microRNA, and biosimilars of these biopharmaceuticals. But the biopharmaceutical is not limited to the above-described ones.

In the prefilled syringe 1 of this embodiment, the outer cylinder 2 has the open distal-end portion which is sealed with the sealing member (seal cap) 3 removably mounted on the outer cylinder. As shown in Figs. 3 and 4, the sealing member (seal cap) 3 has a closed end, a tubular body part, and the nozzle accommodation part formed inside the tubular part. The nozzle accommodation part has a portion accommodating the distal-end portion of the nozzle body portion 24 and a portion accommodating the distal-end portion of the collar 25.

The tubular body part is a cylindrical part whose upper end is closed and whose lower end is formed as an opening. The nozzle accommodation part accommodates almost the entire nozzle part 22. The nozzle accommodation part has a short tubular portion formed downward (toward open portion) from the inner side of the closed end and concentrically with the tubular body part. A rib engageable with the threadedly engaging portion 26 formed on the inner surface of the collar 25 is formed at a lower-end portion of the short tubular part.

An antiskid projected portion is formed on an outer surface of the cap. A plurality of ribs is formed not only on an upper surface of the closed end but also on a lower surface of the tubular body part.

As materials for forming the sealing member (seal cap), it is preferable to use elastic materials such as natural rubber, isoprene rubber, butyl rubber, butadiene rubber, fluoro-rubber, synthetic rubber such as silicone rubber, and thermoplastic elastomers such as an olefin-based elastomer, a styrene-based elastomer, and the like.

The prefilled syringe 1 of the present invention is sterilized by radioactive rays or electron beams with the liquid for medical use being sealed inside the outer cylinder 2. It is preferable to sterilize the prefilled syringe by electron beams.

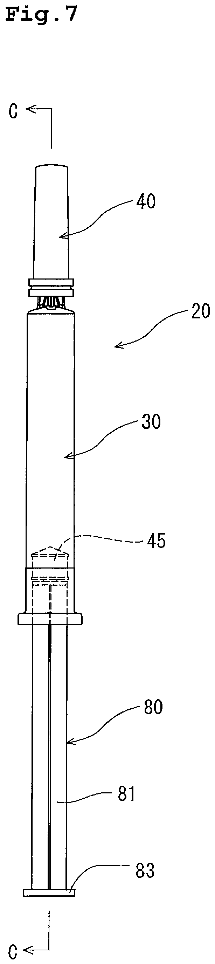

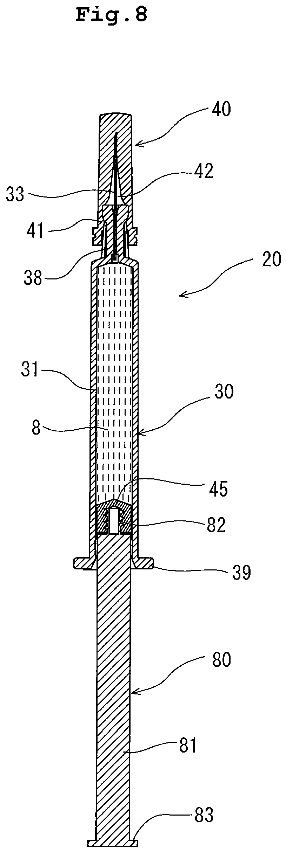

In all of the above-described embodiments, although the outer cylinder 2 has the distal-end open portion, the outer cylinder is not limited to such a form. An outer cylinder 30 of a needle-attached type as shown in Figs. 7 and 8 may be used as the outer cylinder, syringe, and prefilled syringe of the present invention.

A prefilled syringe 20 shown in Figs. 7 and 8 has a needle-attached outer cylinder 30 having a needle tube 33, a sealing member (cap) 40 mounted on a distal-end portion (needle portion) of the outer cylinder 30, a gasket 45 accommodated inside the outer cylinder 30 and being slidable inside the outer cylinder, a plunger 80 mounted on the gasket 45, and the above-described liquid 8 for medical use filled inside the outer cylinder 30.

The needle tube 33 having an outer diameter of ϕ0.41 to 0.18mm is used. The needle tube 33 has a lumen penetrating therethrough from its distal end to proximal end. The needle tube 33 has a needle tip to be punctured into a living body at its distal end. The needle tip having a blade surface is formed at an acute angle. A distal-end portion of the needle tube 33 including the needle tip projects from the distal end of a distal-end portion 38 of the outer cylinder 30. A proximal end of the needle tube 33 penetrates through a needle insertion hole, thus reaching the inside of the outer cylinder 30.

As materials for the metal needle tube 33, stainless steel is preferable. The materials therefor are not limited to stainless steel, but it is possible to use aluminum, aluminum alloys, titanium, titanium alloys, and other metals. As the needle tube 33, it is possible to use not only a straight needle conforming to the ISO standard, but also a tapered needle, a portion of which is tapered.

The outer cylinder 30 has a body part 31 in which a medical agent is filled and a distal-end part 38 having the needle insertion hole. The body part 31 has an internal accommodation portion and is formed substantially cylindrically. A flange 39 is formed at an axial proximal-end side of the body part 31.

The distal-end part 38 has a distal bulged portion and a tubular portion connecting the distal bulged portion and the distal end of the body part 31 to each other. The distal-end part 38 has the needle insertion hole penetrating therethrough. The needle insertion hole is provided with a proximal end of the needle tube 33 and is formed integrally with the outer cylinder by insert molding or the like.

The cap 40 is formed cylindrically, open at a proximal-end side thereof in its axial direction, and closed at a distal end thereof in its axial direction. The cap 40 is formed of an elastic member such as rubber and an elastomer and the like. The cap 40 is mounted on the distal-end part 38 of the outer cylinder 30 in such a way as to cover the needle tip of the needle tube 33 and the distal-end part 38 of the outer cylinder 30. As shown in Fig. 8, the needle tube 33 and the distal-end part 38 are inserted into a lumen 42 of the cap 40.

The inner diameter of the lumen of the cap 40 is formed almost equally to the outer diameter of the distal bulged portion of the distal-end part or a little smaller than the outer diameter of the distal bulged portion. Therefore, when the cap 40 is mounted on the distal-end part 38, the outer peripheral surface of the distal bulged portion closely contacts the inner peripheral surface of the cap 40. Thereby a space covering the needle tube 33 projected from the outer cylinder 30 is closed with the distal bulged portion and the inner peripheral surface of the cap 40. This construction is capable of preventing bacteria from sticking to the needle tip.

An annular rib 41 formed on the inner peripheral surface of the cap 40 tightens a constricted portion disposed at the boundary between the distal bulged portion of the distal-end part 38 and a tapered fitting portion by an elastic force of the cap. Owing to the engagement between the inner peripheral surface of the cap 40 and the constricted portion of the distal-end part 38, it is possible to prevent the cap 40 from being removed from the distal-end part 38 during delivery.

The plunger 80 has a body part 81, a gasket-mounting part 82 formed at a distal end of the body part 81, and a pressing portion 83 provided at a proximal-end portion of the body part. The gasket has a plunger-mounting part receiving and engaging the gasket-mounting part 82 of the plunger 80.

The outer cylinder 30 of this embodiment is also formed of the above-described synthetic resin except the metal needle tube 33.

A liquid-filled sterilized synthetic-resin container 10 of the present invention is composed of a synthetic-resin container body 6, a sealing member 7 sealing an open portion of the container body, and the liquid 8 for medical use accommodated inside the container body. The container is sterilized by radioactive rays or electron beams. The synthetic-resin container body 6 contains the phenol-based antioxidant. The amount of the phenoxyl radicals of the synthetic-resin container measured by the electron spin resonance apparatus is 0.1 to 1.0 × 10¹² spins/mg. The liquid 8 for medical use contains substances, for medical use, which undergo oxidative denaturation.

The container 10 made of synthetic resin is composed of the synthetic-resin container body 6 made of synthetic resin, the sealing member 7 sealing the open portion of the container body 6, and the liquid 8 for medical use accommodated inside the container body. As materials for forming the container body 6, it is possible to preferably use those for forming the outer cylinder 2 made of synthetic resin, as described previously. As the liquid 8 for medical use accommodated inside the container body, it is possible to preferably use the liquid 72 for medical use to be used for the above-described prefilled syringe.

The medical agent-accommodated container 10 of the present invention has the medical agent container body 6 having the open portion, the sealing member (rubber plug in this embodiment) 7 mounted on the open portion of the medical agent container body 6 and sealing the open portion, and the liquid 8 for medical use accommodated inside the medical agent container body 6.

The medical agent container body 6 may have any configuration so long as it has the open portion and is capable of accommodating the liquid for medical use therein. The container body 6 of this embodiment has a cylindrical body part 61 whose lower end is closed, an open portion 62 having a thick flange, and a small-diameter neck portion 63, formed between the open portion 62 and the body part 61, which has a smaller diameter than other portions. A portion between the open portion 62 of the medical agent container body 6 and the neck portion 63 is formed as an accommodation portion accommodating an entry portion 4, of the rubber plug 7, which enters the container and is extended in an equal diameter.

Materials for forming the outer cylinder 2 made of synthetic resin can be preferably used as materials for forming the container body 6. The liquid 8 for medical use which is accommodated inside the prefilled syringe can be preferably used as the liquid 8 for medical use to be accommodated inside the container body.

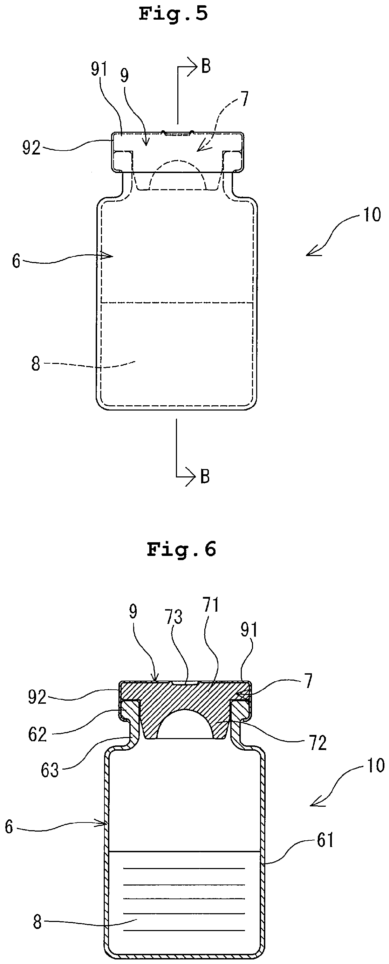

As shown in Figs. 5 and 6, the rubber plug 7 which is the sealing member has a disk-shaped body part 71 and the entry portion 72 which enters the container and is extended downward in a diameter smaller than an outer diameter of the body part 71 from the center of a lower surface of the body part 71. The peripheral portion of the lower surface of the body part 71 constitutes an annular contact portion which contacts an upper surface of the open portion of the container body 6. The entry portion 72 which enters the container has a tubular portion extended in a substantially equal outer diameter and a diameter-decreased tapered portion provided at a distal end of the tubular portion. An outer circumferential surface of the entry portion 72 contacts an inner circumferential surface of the open portion 62 of the medical agent container body 6, thus forming a liquid-tight state. An annular rib and a concave portion 73 formed inside the annular rib are formed on an upper surface of the body part 71.

The configuration of the rubber plug shown in the drawings is an example. It is possible to use any configuration capable of liquid-tightly sealing the open portion 62 of the medical agent container 6. For example, although the above-described rubber plug has the entry portion 72 which enters the container, it is possible to use a rubber plug not having the entry portion 72 which enters the container but having a tubular part covering the outside of the open portion 62 of the container body 6.

As materials for composing the rubber plug 7, elastic materials are preferable. Although the elastic materials are not limited to specific ones, various rubber materials (those subjected to vulcanization are preferable) such as natural rubber, isoprene rubber, butyl rubber, chloroprene rubber, nitrile-butadiene rubber, styrene-butadiene rubber, silicone rubber, and the like are exemplified. Diene rubber is especially preferable from the standpoint that it has an elastic property and can be sterilized by γ rays, electron beams or highpressure steam.

The medical agent-accommodated medical agent container 10 of this embodiment has a covering member 9 on which the rubber plug 7 has been mounted and which covers the peripheral portion of the open portion 62 of the medical agent container body 6 and the peripheral portion of the rubber plug 7. It is preferable that the covering member 9 is formed of aluminum, a heat-shrinkable film or the like and is in close contact with the rubber plug and the medical agent container body. It is possible to use the covering member 9 covering the entire upper surface of the rubber plug 7 so long as a piercing needle such as an injection needle can be pierced thereinto. In this embodiment, the covering member 9 has an annular part 92 and a thin disk-shaped upper surface part 91. A lower-end portion of the annular portion 92 covers an annular lower surface of the open flange portion 62 of the container body 61.

The pressure inside the medical agent container 10 may be decreased.

### EXAMPLES

### Example 1

0.002 wt% of tetrakis [methylene-3-(3,5-di-t-butyl)-4-hydroxyphenyl)propionate] which is a hindered phenol-based antioxidant (trade name: IRGANOX 1010 (produced by BASF Corporation) having a molecular weight of about 1177.7 was added to ZEONEX 480 (trade name) which is cyclic polyolefin [produced by Nippon Zeon Co., Ltd., glass transition temperature: 139 degrees C, MFR: 20g/10 minutes (280 degrees C, load 21N)] to prepare a cyclic polyolefin composition. By using the prepared cyclic polyolefin composition, an outer cylinder having the configuration as shown in Figs. 1 and 2 was prepared. The prepared synthetic-resin outer cylinder was irradiated by electron beams at 25 kGy to sterilize it. In this manner, a sterilized synthetic-resin outer cylinder of the present invention was prepared.

### Example 2

0.0005 wt% of tetrakis [methylene-3-(3,5-di-t-butyl)-4-hydroxyphenyl)propionate] which is a hindered phenol-based antioxidant (trade name: IRGANOX 1010 (produced by BASF Corporation) having a molecular weight of about 1177.7 and 0.1 wt% of tris(2,4-di-t-butylphenyl)phosphite which is a phosphorous-based antioxidant, trade name: IRGAFOS 168, molecular weight: 643.9 (produced by BASF Corporation) were added to the ZEONEX 480 (trade name) which is cyclic polyolefin [produced by Nippon Zeon Co., Ltd., glass transition temperature: 139 degrees C, MFR: 20g/10 minutes (280 degrees C, load 21N)] to prepare the cyclic polyolefin composition. By using the prepared cyclic polyolefin composition, an outer cylinder having the configuration as shown in Figs. 1 and 2 was prepared. The prepared synthetic-resin outer cylinder was irradiated by electron beams at 25 kGy to sterilize it. In this manner, a sterilized synthetic-resin outer cylinder of the present invention was prepared.

### Example 3

Except that the hindered phenol-based antioxidant was added to the cyclic polyolefin at 0.001 wt% which was smaller than the weight percentage of the hindered phenol-based antioxidant of the example 1 to prepare the cyclic polyolefin composition. By using the prepared cyclic polyolefin composition, an outer cylinder having the configuration as shown in Figs. 1 and 2 was prepared. The prepared synthetic-resin outer cylinder was irradiated by electron beams at 25 kGy to sterilize it. In this manner, a synthetic-resin sterilized outer cylinder of the example was produced.

### Comparison Example 1

Except that the hindered phenol-based antioxidant which was added to the cyclic polyolefin at 0.1 wt% which was larger than the weight percentage of the hindered phenol-based antioxidant of the example 1 to prepare the cyclic polyolefin composition. By using the prepared cyclic polyolefin composition, an outer cylinder having the configuration as shown in Figs. 1 and 2 was prepared. The prepared synthetic-resin outer cylinder was irradiated by electron beams at 25 kGy to sterilize it. In this manner, a synthetic-resin sterilized outer cylinder of the comparison example was produced.

### Example 4

A butyl rubber-made gasket for a syringe, having a configuration shown in Figs. 3 and 4 was prepared. A plunger made of polypropylene was prepared. By using the unsterilized synthetic-resin outer cylinder of the example 1, the above-described gasket, and a seal cap, a syringe having a capacity of 1mL was prepared. Another seal cap made of butyl rubber as shown in Figs. 3 and 4 was prepared.

Erythropoietin (produced by Sigma-Aldrich Corporation) was added to an aqueous solution containing 2mM of Na₂HPO₄ and 0.06mg/mL of polysorbate 80 and completely dissolved therein to prepare a solution (erythropoietin solution formulation) in which the concentration of the erythropoietin was 24,000IU/mL.

After 1mL of the erythropoietin solution formulation was filled inside the above-described syringe, the distal-end opening of the outer cylinder was sealed with the seal cap made of butyl rubber.

The medical agent-filled syringe prepared in this manner was irradiated by electron beams at 25 kGy to sterilize the syringe. In this manner, a sterilized prefilled syringe of the example was produced.

### Example 5

Except that the unsterilized synthetic-resin outer cylinder of the example 2 was used, the sterilized prefilled syringe of the example was produced by performing an operation in a manner similar to that of the example 3.

### Example 6

Except that the unsterilized synthetic-resin outer cylinder of the example 3 was used, the sterilized prefilled syringe of the example was produced by performing an operation in a manner similar to that of the example 3.

### Comparison Example 2

Except that the unsterilized synthetic-resin outer cylinder of the comparison example 1 was used, the sterilized prefilled syringe of the comparison example was produced by performing an operation in a manner similar to that of the example 3.

### Experiment 1

The amount of radicals contained in the sterilized synthetic-resin outer cylinder of each of the examples 1, 2 and the comparison example 1 was measured.

By cutting off specimens from the sterilized outer cylinders made of synthetic resin and using an electron spin resonance apparatus ("E500" produced by Bruker Corporation), the amount of the radicals of each of the above-described outer cylinders was measured at room temperature (25 degrees C) under the following conditions:
▪ Microwave frequency: approximately 9.9 GHz
▪ Microwave intensity: 0.025 to 2.0 mW
▪ Sweep magnetic field: 40 mT (400 Gauss)
▪ Range of modulated magnetic field: 0.1 mT (1.0 Gauss)
▪ Standard specimen: 1,1-diphenyl-2-picrylhydrazyl (DPPH).

By using a calibration curve prepared from ESR spectra of standard specimens (DPPH) having known concentrations, the amount (spins/mg) of the phenoxyl radicals per 1mg of each specimen was found. The results were as shown in table 1.

**Table 1**

| | Amount of radical (× 10¹² spins/mg) |
|---|---|
| Example 1 | 0.30 |
| Example 2 | 0.04 |
| Example 3 | 0.16 |
| Comparison example 1 | 28 |

### Experiment 2

After three sterilized prefilled syringes prepared in the examples 3, 4 and the comparison example 2 were preserved for four weeks in a condition where the temperature was 25 degrees C and the humidity was 60% RH, the oxidation rate of a methionine residue in the erythropoietin contained in an erythropoietin solution formulation accommodated inside each syringe was measured by the following method. An average value of three specimens of each of the examples 3, 4 and the comparison example 2 was taken to calculate the oxidation rate (%) of each methionine residue.
(i) The mixture of the 100µL of the erythropoietin solution formulation accommodated inside each prefilled syringe and 400µL (100mM, pH: 8.0) of ammonium acetate was placed on a saucer of a centrifugal filtration device (Amicon Ultra-0.5 10K produced by Milipore Ireland Ltd.,) to centrifuge the mixture at 14,000G for 15 minutes.
(ii) After the residue on each filter paper was collected, an ammonium acetate solution (100mM, pH:8.0) was added to each collected residue in such a way that the whole amount of the solution was 50µL. 1µg/mL of Glu-C (pH: 5.6) and 100mM of ammonium acetate were added to the solution to separate an oxidized methionine fragment and an unoxidized methionine fragment from each other.
(iii) After the specimens were incubated at 37 degrees C for 24 hours, each specimen was diluted with 10mM of the ammonium acetate solution (pH: 8.0) to subject the specimens to high-performance liquid chromatography analysis (HPLC). As a result of comparison between the oxidation rates by methionine residue of the examples and those of the comparison examples, it could be confirmed that the former was superior to the latter.

### Condition of HPLC

▪ Mobile phase A: aqueous solution of 0.05% trifluoroacetic acid
▪ Mobile phase B: acetonitrile containing 0.05% trifluoroacetic acid
▪ Column: octadecyl group-bonded silica gel column (Inertsil ODS-3)(5µm, 205mm × 2.1mm i.d.)
▪ Temperature of column: 40 degrees C
▪ Injection amount: 30µL
▪ Total flow rate: 0.25mL/minute
▪ Flow mode: linear gradient mode
▪ Measurement wavelength: 280nm

### INDUSTRIAL APPLICABILITY

The synthetic-resin outer cylinder of the present invention for the syringe has the following form:
(1) A synthetic-resin outer cylinder for a syringe which is sterilized by radioactive rays or electron beams, said synthetic-resin outer cylinder having an open distal-end portion or an injection needle fixed to a distal-end portion thereof, wherein said synthetic resin contains a phenol-based antioxidant; and an amount of phenoxyl radicals of said synthetic resin measured by an electron spin resonance apparatus is 0.1 to 1.0 × 10¹² spins/mg.

Although the synthetic-resin outer cylinder of the present invention for the syringe contains the phenol-based antioxidant and is sterilized by radioactive rays or electron beams, a medical agent filled inside the outer cylinder is oxidatively denatured to a very low extent.

The above-described embodiment may have the following form:
(2) A synthetic-resin outer cylinder according to the above (1), wherein said synthetic resin is olefin-based resin.
(3) A synthetic-resin outer cylinder according to the above (1) or (2), wherein said synthetic resin is cyclic polyolefin.
(4) A synthetic-resin outer cylinder according to any one of the above (1) through (3), wherein said synthetic resin contains an antioxidant not phenol-based in addition to said phenol-based antioxidant.

The syringe of the present invention has the following form:
(5) A syringe comprising a synthetic-resin outer cylinder according to any one of the above (1) through (4), a gasket slidable inside said outer cylinder, and a plunger mounted on said gasket; said syringe being sterilized by radioactive rays or electron beams together with said outer cylinder.

The prefilled syringe of the present invention has the following form:
(6) A prefilled syringe comprising a synthetic-resin outer cylinder which has an open distal-end portion or an injection needle fixed to a distal portion thereof, a gasket slidable inside said outer cylinder, a plunger mounting on said gasket, a sealing member sealing said open distal-end portion of said synthetic-resin outer cylinder or said injection needle, and a liquid for medical use filled inside said outer cylinder; said prefilled syringe being sterilized by radial rays or electron beams, wherein said synthetic-resin outer cylinder contains a phenol-based antioxidant; an amount of phenoxyl radicals of said synthetic-resin outer cylinder measured by an electron spin resonance apparatus is 0.1 to 1.0 × 10¹² spins/mg; and said liquid for medical use contains substances, for medical use, which undergo oxidative denaturation.

The outer cylinder of the prefilled syringe of the present invention is made of the above-described resin. Thus, although the prefilled syringe is sterilized by radioactive rays or electron beams, a medical agent filled inside the prefilled syringe is oxidatively denatured to a very low extent.

The above-described embodiment may have the following form:
(7) A prefilled syringe according to the above (6), wherein said liquid for medical use is a protein solution formulation.
(8) A prefilled syringe according to the above (6) or (7), wherein said synthetic resin is olefin-based resin.
(9) A prefilled syringe according to the above (6) or (7), wherein said synthetic resin is cyclic polyolefin.
(10) A prefilled syringe according to any one of the above (6) through (9), wherein said synthetic resin contains an antioxidant not phenol-based in addition to said phenol-based antioxidant.

The liquid-filled sterilized synthetic-resin container of the present invention has the following form:
(11) A liquid-filled sterilized synthetic-resin container comprising a synthetic-resin container body, a sealing member sealing an open portion of said container body, and a liquid for medical use accommodated inside said container body; said synthetic-resin container being sterilized by radioactive rays or electron beams, wherein said synthetic-resin container body contains a phenol-based antioxidant; an amount of phenoxyl radicals of said synthetic-resin container body measured by an electron spin resonance apparatus is 0.1 to 1.0 × 10¹² spins/mg; and said liquid for medical use contains substances, for medical use, which undergo oxidative denaturation.

The resin container body containing the phenol-based antioxidant and the phenoxyl radicals in the amount of 0.1 to 1.0 × 10¹² spins/mg measured by the electron spin resonance apparatus is used for the liquid-filled sterilized synthetic-resin container of the present invention. Thus, although the synthetic-resin container is sterilized by radioactive rays or electron beams, a medical agent filled inside the container body is oxidatively denatured to a very low extent.

The above-described embodiment may have the following form:
(12) A liquid-filled sterilized synthetic-resin container according to the above (11), wherein said liquid for medical use is a protein solution formulation.
(13) A liquid-filled sterilized synthetic-resin container according to the above (11) or (12), wherein said synthetic resin is olefin-based resin.
(14) A liquid-filled sterilized synthetic-resin container according to the above (11) or (12), wherein said synthetic resin is cyclic polyolefin.
(15) A liquid-filled sterilized synthetic-resin container according to any one of the above (11) through (14), wherein said synthetic resin contains an antioxidant not phenol-based in addition to said phenol-based antioxidant.

## Claims

1. A synthetic-resin outer cylinder for a syringe which is sterilized by radioactive rays or electron beams,
said synthetic-resin outer cylinder having an open distal-end portion or an injection needle fixed to a distal-end portion thereof,
wherein said synthetic resin contains a phenol-based antioxidant; and an amount of phenoxyl radicals of said synthetic resin measured by an electron spin resonance apparatus is 0.1 to 1.0 × 10¹² spins/mg.

2. A synthetic-resin outer cylinder according to claim 1, wherein said synthetic resin is olefin-based resin.

3. A synthetic-resin outer cylinder according to claim 1 or 2, wherein said synthetic resin is cyclic polyolefin.

4. A synthetic-resin outer cylinder according to any one of claims 1 through 3, wherein said synthetic resin contains an antioxidant not phenol-based in addition to said phenol-based antioxidant.

5. A syringe comprising a synthetic-resin outer cylinder according to any one of claims 1 through 4, a gasket slidable inside said outer cylinder, and a plunger mounted on said gasket; said syringe being sterilized by radioactive rays or electron beams together with said outer cylinder.

6. A prefilled syringe comprising a synthetic-resin outer cylinder which has an open distal-end portion or an injection needle fixed to a distal portion thereof, a gasket slidable inside said outer cylinder, a plunger mounting on said gasket, a sealing member sealing said open distal-end portion of said synthetic-resin outer cylinder or said injection needle, and a liquid for medical use filled inside said outer cylinder; said prefilled syringe being sterilized by radial rays or electron beams,
wherein said synthetic-resin outer cylinder contains a phenol-based antioxidant; an amount of phenoxyl radicals of said synthetic-resin outer cylinder measured by an electron spin resonance apparatus is 0.1 to 1.0 × 10¹² spins/mg; and said liquid for medical use contains substances, for medical use, which undergo oxidative denaturation.

7. A prefilled syringe according to claim 6, wherein said liquid for medical use is a protein solution formulation.

8. A prefilled syringe according to claim 6 or 7, wherein said synthetic resin is olefin-based resin.

9. A prefilled syringe according to claim 6 or 7, wherein said synthetic resin is cyclic polyolefin.

10. A prefilled syringe according to any one of claims 6 through 9, wherein said synthetic resin contains an antioxidant not phenol-based in addition to said phenol-based antioxidant.

11. A liquid-filled sterilized synthetic-resin container comprising a synthetic-resin container body, a sealing member sealing an open portion of said container body, and a liquid for medical use accommodated inside said container body; said synthetic-resin container being sterilized by radioactive rays or electron beams,
wherein said synthetic-resin container body contains a phenol-based antioxidant; an amount of phenoxyl radicals of said synthetic-resin container body measured by an electron spin resonance apparatus is 0.1 to 1.0 × 10¹² spins/mg; and said liquid for medical use contains substances, for medical use, which undergo oxidative denaturation.

12. A liquid-filled sterilized synthetic-resin container according to claim 11, wherein said liquid for medical use is a protein solution formulation.

13. A liquid-filled sterilized synthetic-resin container according to claim 11 or 12, wherein said synthetic resin is olefin-based resin.

14. A liquid-filled sterilized synthetic-resin container according to claim 11 or 12, wherein said synthetic resin is cyclic polyolefin.

15. A liquid-filled sterilized synthetic-resin container according to any one of claims 11 through 14, wherein said synthetic resin contains an antioxidant not phenol-based in addition to said phenol-based antioxidant.
